# EUROPEAN PATENT SPECIFICATION

(11) **EP 1 596 872 B1**
(45) Date of publication and mention of the grant of the patent: **22.06.2011**
(21) Application number: 04704822.8
(22) Date of filing: 23.01.2004
(51) Int. Cl.: A61K 31/7048, A61K 35/74

(54) **COMPOSITIONS AND METHODS FOR RESTORING BACTERIAL FLORA**
ZUSAMMENSETZUNGEN UND VERFAHREN ZUR WIEDERHERSTELLUNG DER BAKTERIENFLORA
COMPOSITIONS ET PROCEDES POUR RESTAURER LA FLORE BACTERIENNE

(30) Priority: 24.01.2003 US 442238 P
(43) Date of publication of application: 23.11.2005
(73) Proprietor: Flora Technology Inc., Aberdeen, Washington 98520 (US)
(72) Inventor: DRAKE, Pamela, M., Aberdeen, Washington 98520 (US); STOPSEN, Jessie, A., Montesano, WA 98563 (US)
(74) Representative: Viering, Jentschura & Partner
(86) International application number: PCT/US2004/001775
(87) International publication number: WO 2004/067013

(56) References cited:
- EP-A- 1 195 095
- WO-A-97/29763
- GB-A- 2 369 777
- US-B1- 6 461 607
- PATENT ABSTRACTS OF JAPAN vol. 1999, no. 03, 31 March 1999 (1999-03-31) & JP 10 330267 A (MEIJI SEIKA KAISHA LTD), 15 December 1998 (1998-12-15)

## Description

### BACKGROUND OF THE INVENTION

### Field of the Invention

An integral part of the human digestive system is the delicate intestinal environment and the equally delicate balance of bacteria that are essential to proper nutrition and health of the gastric system. The intestinal flora is made up of many different types of living bacteria that have a symbiotic relationship with the rest of the body. At least 400 different friendly species exist, including *L. acidophilus* and *B. bifidus.* Friendly bacteria provide a number of benefits, including enhancing digestion and nutrient absorption, improving bowel function and supporting natural immunity. In additional, friendly bacteria produce vitamins and other nutrients and assist in the digestion of proteins and sugars. Another important role of friendly bacteria is inhibiting the growth of pathogenic bacteria and other microbes, including viruses and protozoa. Friendly bacteria can inhibit pathogenic microorganisms in a number of ways, including secreting substances that reduce the pH of the gastrointestinal and genitourinary tracts, thereby making them less hospitable to pathogenic microorganisms, and secreting bacteriocins, which kill pathogenic bacteria.

Disruption of normal bacterial flora can result in infection by a variety of pathogenic microorganisms, including bacteria, viruses, and protozoa, and result in disease and other disorders. Increasingly in modern times, the delicate balance of bacteria has come under attack from several environmental factors. Unfortunately, the most common type of friendly bacteria found in the digestive system of those over two years of age is the type of bacteria that is most easily damaged by today's living environment. The most potent damaging environmental factors are the use of antibiotics to combat disease and the routine use of antibiotics in our food supply. When the antibiotics destroy the bacteria that cause disease, the friendly bacteria in our digestive system are also frequently destroyed. In addition, many meat products, dairy products, and fruits contain antibiotics to enhance growth. This also damages the bacteria in the human digestive system. The use of alcohol as an intoxicating beverage also takes it toll on the desirable bacteria. Chlorine and fluoride in our municipal water supply further contribute to compromise the gut flora. In addition, bacteria are living organisms and are, therefore, susceptible to damage from most pharmaceutical drugs, including chemotherapeutic agents, antibiotics, hormones, immunizations and antacids, and radiation.

The disruption of normal bacterial flora can lead to superinfection by pathogenic microorganisms. It is interesting to note that the rampant cases of E. Coli and super germ mutations have much of their origin in the gut because the majority of the population does not have the needed delicate balance of bacteria to prevent such sickness. Superinfection is relatively common and potentially very dangerous, because the pathogenic microorganisms responsible for the infection are, in many cases, Enterobacteriaceae, Pseudomonas, or Candida or other fungi, which may be difficult to eradicate with presently available antiinfective drugs. Superinfection frequently results from the disruption of normal friendly bacteria, particularly following treatment with antibiotics. The more "broad" the effect of an antibiotic on microorganisms, the greater the alteration in the normal microflora and the greater the possibility that one or more pathogenic microorganisms will become predominant, invade the host, and produce infection. It is expected that further development and use of broad-spectrum agents will lead to more extensive adorations in the normal flora and, thus, more superinfections. Consequently, there is a recognized need in the art for compositions and methods to maintain and restore normal bacterial flora, particularly during or following treatment with antibiotics, drugs, or radiation, and in immunocompromised subjects.

### Description of the Related Art

Current approaches to maintaining and/or restoring normal bacterial flora include the use of friendly bacteria-laden products, including supplements comprising friendly bacterial strains, such as Lactobacillus acidophilus. The most common lay approach today is to eat yogurt or drink Acidophilus milk. However, the viability of the strains present in these supplements and food products is questionable. For example, the bacteria used may be weak strains that cannot endure intestinal environmental conditions. Furthermore, bacteria as an organism has its own nutritional and environmental requirements for its viability and health, and these may not be present or optimal in the digestive or genitourinary tracts of subjects.

### BRIEF SUMMARY OF THE INVENTION

The present invention relates generally to kits comprising beneficial bacteria, and, bacteria nutrients to support bacteria growth and implantation and an antifungal agent, such as nystatin, to reduce levels of pathological organisms in the subject.

In one embodiment, the invention provides a kit that includes a bacteria, a bacteria nutrient, and an antimicrobial agent, wherein the antimicrobial agent is an antifungal compound. In one specific embodiment, the antifungal compound is nystatin. The bacteria is Lactobacillus acidophilus, Lactobacillus bulgaricus, Streptococcus thermophilous, Bifodophilus longum, Bifidobacteria bifidus, Bacillus laterosporus, Bacillus bifidum, Lactobacillus plantaterum, Lactobacillus rueteri, or Lactobacillus salivarus. In one embodiment, the bacteria is prepared by filtration. The bacteria nutrient is selected from the group consisting of spirilinal and/or a blend of high lipid chloraphylling, fructooligosaccharides, and or inulins. The kit further includes a methylsulfonylmethane mixture, said mixture comprising methylsulfonylmethane and ascorbic acid.

In one embodiment, the invention provides a kit, wherein the bacteria include Lactobacillus acidophilus, Lactobacillus bulgaricus, and Streptococcus thermophilus.

In a related embodiment, the invention provides a kit, wherein the bacteria include Lactobacillus acidophilus, Streptococcus thermophilus, Lactobacillus bulgaricus, Bifodophilus longum, Bifidobacteria bifidus, and Bacillus laterosporus.

In another related embodiment, the invention provides a kit, wherein the bacteria include Lactobacillus acidophilus, Bacillus bifidum, Lactobacillus bulgaricus, Lactobacillus plantarum, Lactobacillus rueteri, Streptococcus thermophilus, Lactobacillus salivarus, and Bifidobacteria bifidus.

In yet another related embodiment, the invention provides a kit that includes Bifodophilus infantis and fructooligosaccharides. In certain embodiments, these components are in powder form.

The invention further provides a related kit, wherein the bacteria include Lactobacillus acidophilus, Bacillus bifidum, Lactobacillus bulgaricus, Lactobacillus planterum, Lactobacillus rueteri, Streptococcus thermophilus, Lactobacillus sativarius, and Bifidobacteria bifidus.

In another embodiment, the invention includes the use of the kits/compositions for the manufacture of a pharmaceutical for enhancing bacteria in a patient. In certain embodiment, the patient is a human or a non-human animal. In specific embodiments of the methods of the invention, the bacteria and bacteria nutrient are introduced orally, rectally, or vaginally. The antimicrobial agent is an antifungal compound. In a specific embodiment, the antifungal compound is nystatin. In other related embodiments, the patient is also being treated with an antibiotic, radiation, a hormone, chemotherapy, ulcer medication, a steroid, or a vaccine.

In another related embodiment, the patient is also being treated with chemotherapy.

In yet another related embodiment, the patient is also being treated with radiation.

In a further related embodiment of the invention, the patient is also being treated with an antibiotic.

In another embodiment, the invention provides for the use of the inventive kits/compositions for the manufacture of a pharmaceutical for inhibiting or reducing infection in a patient. In specific embodiments of the method, the patient is suffering from cancer, acquired immunodeficiency disease, autoimmune disorders, food allergy, lactose intolerance, fatigue, malnutrition, neuropsychiatric symptoms, inflammatory bowel disease, ulcerative colitis, diarrheal diseases, diverticulitis, ostomies, ulcers, high cholesterol, chronic disease, immunocompromization, bacterial infection, yeast infection, viral infection, fungal infection, irritable bowel syndrome, Krohn's disease, periodontal disease, chronic respiratory or upper respiratory infection, ear infection, sinus infection, necrotizing enterocolitis, ileocecitis, multiple organ failure syndrome, pancreatitis, or burn injury.

### DETAILED DESCRIPTION OF THE INVENTION

The present invention provides an integrated approach to restoring and maintaining beneficial bacteria in a subject by providing compositions and kits comprising beneficial bacteria, and bacteria nutrients to support bacteria growth and implantation and an antifungal agent, such as nystatin, to reduce levels of pathological organisms in the subject. Thus, the invention kills off harmful microorganisms, such as yeast, for example, while supporting the growth and establishment of friendly bacteria.

The invention typically is prepared in a kit, which offers both ease of prescription by a medical professional and ease of use by a patient. Such kits, therefore, fulfill a need in the medical field. Typically, such kits may include a component including the bacteria and bacteria nutrients and a component including the antifungal agent, although, in certain embodiment, these components may be combined. Compositions, kits and kit components, as well related methods are described in further detail infra.

### A. Probiotic Compositions

The invention provides compositions useful for restoring friendly or beneficial bacteria to a subject. Thus, in certain embodiments, the invention provides compositions, mixtures, formulations, or kits that include such bacteria. These compositions and kits also include food or nutrients to promote growth and proliferation of the bacteria in the subject. In addition, an antifungal agent is included to reduce the presence of undesirable or pathogenic microbes, such as yeast, for example, in the subject.

### 1. Beneficial Bacteria

It is estimated that over 400 different species of beneficial bacteria exist in the human gastrointestinal tract, and any of these species may be used according to the invention. Compositions of the invention may contain a single species or strain of bacteria, or they may comprise two or more strains or species of bacteria. In certain embodiments, compositions of the invention include a variety of different bacteria or strains.

The choice of bacteria may be selected based on a number of different criteria, including, for example, clinical identification of strains lacking from a subject or identification of a microorganism superinfecting the subject. Studies have demonstrated that certain beneficial bacteria are more effective than others in the treatment or protocol of certain disease or to combat superinfection by specific microbes and have clearly established the therapeutic utility of specific bacteria. For example, Bifodophilus longum has been shown to be effective in preventing bowel cancer. Lactobacillus has been shown to assist in the metabolism of drugs by the gut flora (Eur. J. Drug Metab. Pharmacokinet 19(3): 201-7 (1994)). Lactobacillus casei sp. Strain GG) promote has been demonstrated to promote recovery from acute diarrhea in children (Pediatrics 88, 90-97). Lactobacillus Acidophilus has prophylactic as well as antibiotic properties (Cultured Dairy Products J. 11 (4); 14-17, Cultured Dairy Products J. 12 (2); 8-11, Cultured Dairy Products J. 18 (2); 15-19, US Patent 3689, 640 Sept. 5). Lactobacillus Acidophilus has been shown to help reduce the occurrence of diarrhea and urinary and vaginal infections (J. Appli. Nutri. 401; 32-43, J. Dairy Science 71; 3222-3228, Infect. Immun. 47: 84-89). Lactobacillus Rhamnosus has been shown to prevent disorders such as lactose intolerance, viral and bacterial diarrhea, food allergy, and inflammatory bowel disease (Appl. Environmental Microbiology Jan. 1999). In addition, Lactobacillus Acidophilus prevents radiotherapy-associated diarrhea (Clinical Radiology 39, 433-439 (1988)). Other studies established the inhibitory effects of Bifidobacterium longum on colon, mammary and liver carcinogenesis induced by 2-amino-3-methylimidayol (4,5-f) guinoline, or food mutagens (Cancer Research 53, 3914-3918). Lactobacillus Plantarum has been associated with a reduction of cardiovascular disease risk factors and could be useful as a protective agent in the primary prevention of atherosclerosis in smokers (AMJ. Clin. Nutri. 76 (6) 49-55 (2002)). Probiotic treatment has also been shown to be beneficial for extensive burn injury (J. Nutr. 125:1401-1412 (1995)).

Specific examples of bacteria that may be included in compositions and kits of the invention include, but are not limited to, Lactobacillus acidophilus, Bifidobacterium bifidus, and Bifodophilus longum. L. acidophilus and B. bifidus are the principal species that have been previously linked with successful rebuilding of friendly gut flora and which are generally available in supplement form. Bacteria present in compositions of the invention are typically Class I bacterial agents as rated by the American Type Culture Collection Catalogue (Rockville, Maryland). These bacteria are indigenous and normal inhabitants of natural soils and freshwater. They are also commonly found on organically grown fruits and vegetables and are considered non-toxic and non-pathogenic. Bacteria that may be used according to the invention include Lactobacillus plantarum, Lactobacillus salivarius, Lactobacillus rueteri, and Lactobacillus bulgaricus, Bifidophilus infantis, Streptococcus thermophilus. Bifidophilus longum, Bifidobacteria bifidus, Bacillus bifidum, and Bacillus laterosporus, for example.

In certain embodiments of the invention, compositions include second or later generation bacterial strains. The use of such strains may offer advantages such as, for example, an increased survival or proliferation rate when introduced to a subject. In one embodiment, a composition of the invention includes the Lactobacillus acidophilus strain DDS 1™ (available from UAS Labs, Minnetonka, MN). Strain DDS 1™ has been shown to be more stable and better able to implant in a subject as compared to first generation strains (Bhatia, Growth optimization of Lactobacillus acidophilus in whey, M.S. Thesis Univ. of Nebraska, Lincoln (1991)).

Embodiments of the invention may also include human strains of bacteria. Human strains are those strains found present in a human. There are advantages associated with using a strain associated with a subject being treated, as described in Praha, Vet Med (1): 19-27 (1997). For example, human strains have been shown to be more viable upon implantation into the human gut as compared to non-human animal strains. The use of human strains is discussed in Golden, B.R. and Gorbach, S.L., Probiotics for humans. In: Probiotics The Scientific Basis (Fuller. R. ed), Ch 13, pp 367-368. Chapman and Hall, London and Salminen, S., Deighton, M. and Gorbach, S. Lactic Acid bacteria in health and disease. In: Lactic Acid Bacteria (Salminen, S. and Wright, A.V ed) Ch. 7, pp 200-201. Marcel Dekker Inc. New York (1993).

In different embodiments, the invention may be bacteria-specific or broad spectrum, depending, in part, on the nature of the disease, disorder, or other reason a subject is being treated. Such specificity is possible, since certain beneficial bacteria have been shown to be more effective than others in combating certain pathological microbes or diseases. Where a specific beneficial bacteria has been identified as able to improve the symptoms of or cure a disease or disorder, the specific bacteria may be used to treat the disease or disorder. However, where a specific beneficial bacteria has not been identified, it may be beneficial to introduce several bacteria. Thus, the invention provides compositions, kits, and uses thereof for combating or improving a disease or disorder wherein the causative agent is unknown.

In certain embodiments of the invention, a genetically altered bacterial strain may be used. Such a strain may be genetically altered in any of a variety of different ways designed to increase efficacy or effectiveness. For example, bacteria may be altered to increase growth or colonization rate. In addition, a bacteria may be altered to produce a beneficial polypeptide, for example, a cytokine or other immune response molecule, an anti-inflammatory molecule, or an apoptosis regulating molecule, active in the subject. It was recently demonstrated that administration of the murine enteric bacterium Lactococcus lactis genetically engineered to produce the anti-inflammatory cytokine, interleukin-10 (IL-10), is therapeutically effective in mouse models of inflammatory bowel disease (discussed in Shanahan, F., Science 289:1311-2 (2000) and references cited within, including Steiler et al., Science 289: 1352 (2000)). Methods of genetically altering bacteria, including the expression of a recombinant polypeptide are widely known and available in the art. Exemplary methods are described in Methods in Cloning Vol. 3, eds. Sambrook and Russell, Cold Spring Harbor Laboratory Press (2001) and references cited within.

Bacteria used according to the invention may be obtained by any available means. A variety of beneficial bacteria are commercially available or available from American Type Culture Collection Catalogue (Rockville, Maryland). Beneficial bacteria may also be cultured, for example, in liquid or on solid media, following routine and established protocols and isolated from the medium by any available means, such as centrifugation or filtration from liquid medium or mechanical removal from solid medium, for example. Exemplary methods are described in Methods in Cloning Vol. 3, eds. Sambrook and Russell, Cold Spring Harbor Laboratory Press (2001) and references cited within. In certain embodiments, one or more of the bacteria included in the composition is isolated or separated from its growth medium by filtration. Isolation by filtration may result in more viable bacteria as compared to bacteria isolated by other methods, particularly centrifugation. Methods of isolating bacteria from medium by filtration are well-known and available in the art. For example, a variety of membrane filters and filtration devices contain filter pore sizes that permit the passage of medium but not bacteria, thereby allowing the isolation of bacteria from the medium.

Compositions and kits of the invention may optionally include a physiologically acceptable carrier. While any suitable carrier known to those of ordinary skill in the art may be employed in the pharmaceutical compositions of this invention, the type of carrier will typically vary depending on the mode of administration. Compositions of the present invention may be formulated for any appropriate manner of administration, including for example, orally and rectally.

Carriers for use within such compositions are biocompatible, and may also be biodegradable. In certain embodiments, the formulation preferably provides a relatively constant level of active component release. In other embodiments, however, a more rapid rate of release immediately upon administration may be desired.

In certain applications, the compositions and kits disclosed herein may be delivered via oral administration to an animal. As such, these compositions may be formulated with an inert diluent or with an assimilable edible carrier, or they may be enclosed in hard- or soft-shell gelatin capsule, or they may be compressed into tablets, or they may be incorporated directly with the food of the diet.

The compositions or certain components thereof may be incorporated with excipients and used in the form of ingestible tablets, buccal tables, troches, capsules, elixirs, suspensions, syrups, wafers, and the like (see, for example, Mathiowitz et al., Nature 1997 Mar 27;386(6623):410-4; Hwang et al., Crit Rev Ther Drug Carrier Syst 1998;15(3):243-84; U. S. Patent 5,641,515; U. S. Patent 5,580,579 and U. S. Patent 5,792,451). Tablets, troches, pills, capsules and the like may also contain any of a variety of additional components, for example, a binder, such as gum tragacanth, acacia, cornstarch, or gelatin; excipients, such as dicalcium phosphate; a disintegrating agent, such as corn starch, potato starch, alginic acid and the like; a lubricant, such as magnesium stearate; and a sweetening agent, such as sucrose, lactose or saccharin may be added or a flavoring agent, such as peppermint, oil of wintergreen, or cherry flavoring. When the dosage unit form is a capsule, it may contain, in addition to materials of the above type, a liquid carrier. Of course, any material used in preparing any dosage unit form should be pharmaceutically pure and substantially non-toxic in the amounts employed. In addition, the active compounds may be incorporated into sustained-release preparation and formulations.

In certain applications, the compositions and kits disclosed herein may be delivered via rectal administration to an animal. As such, these compositions may be formulated with an inert diluent, enclosed in hard- or soft-shell gelatin capsules (*e.g*. vegetarian gel capsules), or they may be present in suppositories, for example. In one embodiment, the kits or certain compositions or components thereof are formulated for delivery in fleet enema form for rectal implantation or administration. In other embodiments, compositions and kits of the invention may be administered rectally via small bowel infusion via a nasoduodenal tube, a gastrostomy, or a colonoscope. Delivery via colonoscope offers the advantages of proximal infusion and the possibility to detect colonic pathology.

The amount of each component present in an inventive composition may be prepared such that a suitable dosage will be obtained in any given unit dose of the composition. Factors such as solubility, bioavailability, biological half-life, route of administration, product shelf life, as well as other pharmacological considerations will be contemplated by one skilled in the art of preparing such compositions and formulations, and, as such, a variety of dosages and treatment regimens may be desirable.

The compositions and kits described herein may be presented in unit-dose or multi-dose containers, such as sealed ampoules or vials. Such containers are typically sealed in such a way to preserve the sterility or stability of the formulation until use. In general, formulations may be stored as powders, solids, suspensions, solutions or emulsions in oily or aqueous vehicles. A composition may also be stored in a freeze-dried condition requiring only the addition of a liquid carrier immediately prior to use.

The skilled artisan would understand that the amount of dosage of bacteria provided to a subject depends on a variety of factors, including the specific bacteria being introduced, the age and weight of the subject, the particular disease or disorder being treated, and whether the bacteria is being provided for therapeutic or maintenance purposes. For example, large-scale replenishment may be appropriate following treatment with an antibiotic, while smaller doses may be appropriate to maintain a healthy level of beneficial bacteria.

Accordingly, dosages vary greatly. In one embodiment, a dosage may range from one billion to 25 billion live organisms. In certain embodiments, dosages for Lactobacillus acidophilus range from 2 billion to 10 billion live organisms. In one embodiment, a supplemental dosage of at least one billion organisms per day is provided to a subject to achieve a critical mass of bacteria restoration following antibiotic treatment. According to the invention, one or more than one strain of bacteria may be introduced to a subject. When more than one bacteria strain is introduced, the amount or dosage of each strain may be a particular ration of one or more strains to another. In certain embodiments, the comparative amount of each bacteria strain introduced may be determined based upon the normal gut flora strains. In certain embodiments of the invention wherein more than one bacteria strain is introduced to a subject and one of the bacteria is Lactobacillus acidophilus, the amount or dosage of the other bacteria may be determined based upon the amount or dosage of Lactobacillus acidophilus. For example, the dosage of Lactobacillus acidophilus may be determined, and the dosage of the other bacteria determined based on the amount of Lactobacillus acidophilus. Again, in certain embodiments, the ratio of different bacteria may be the normal gut flora ratio of the same or similar strains of bacteria.

### 2. Bacteria Food and Nutrients

Compositions and kits of the invention also comprise food and/or nutrient sources for the beneficial bacteria being introduced to a subject. A variety of different nutrients and food sources may be included in the composition or kit. In certain embodiments, compositions of the invention include oligosaccharides or other sugars, including, for example, fructoologosaccharides (FOS). FOS are chain polymers of the sugar fructose that are found in a variety of foods. The sugar units can be linked in a single straight chain or can be a chain with side branches. In many cases, small amounts of glucose are also contained in the chain. Chemically, the length of the fructose chains can vary from source to source. Inulin is an example of a longer chained compound that is considered a FOS. The shorter (lower molecular weight) compounds tend to have a sweet taste.

In certain embodiments, inulins may be included in compositions of the invention. Inulin is a fructooligosaccharide (FOS) derived from chicory by a process similar to that used in extracting sugar from sugar beets. It is white in color, slightly hygroscopic and is water soluble and not digested by monogastric animals. It is not only a natural ingredient but also a prebiotic that stimulates the growth of good intestinal bacteria which support healthy colon conditions. Jerusalem artichoke tubers (JAT) are an excellent source of fructooligosaccharides (FOS). Fresh tubers contain approximately 18-20% solids, of which 70-80% is soluble FOS. Approximately 2/3rds of the FOS have a DP <10 and the remaining carbohydrate is inulin. These ratios can change dramatically during storage and conditions can be manipulated to preserve long chain FOS (Modler et al., 1992a). JAT inulofructosaccharides have been purified. Fructooligosaccharides (FOS) have been shown to stimulate growth of bifidobacteria (Yazawa&Tamura 1982).

A variety of dosages of FOS may be used according to the invention. In one embodiment, the level of FOS intake is 3-7 grams per day of treatment. Maximum FOS intake per day typically does not exceed 18-20 grams for males. In certain embodiments, the intake for males is 0.3 g per kg body weight, and for females it is 0.4 g per kg.

In certain embodiments of the invention, bacteria food and nutrient sources include a combination of spirulin, high lipid chloraphyllins, methylsulfonylmethane (MSM) and, ascorbic acid. Ascorbic acid is added to the MSM for proper utilization in the body. Although any suitable amount or dosage of food may be used, in certain specific embodiments, a chloraphylin dosage is 300 mg, an MSM dosage is 50 mg, and an ascorbic acid dosage is 250 mg.

### 3. Antimicrobials

Compositions of the invention contain one or more anti-fungal agents. Typically, the selected anti-fungal agent inhibits the growth of a fungi.

Compositions and kits of the invention include antifungal agents, typically included to combat yeast infections (*e.g*. Candida albicans) commonly associated with the use of antibiotics or a compromised immune system. In certain embodiments, Nystatin (*i.e*. Mycostatin, Mykinac, Nilstat, Nystex, O-V Statin) is included in compositions of the invention. Nystatin is a common topical treatment for thrush. Nystatin may be used orally or topically. Oral nystatin is also used for treatment of intestinal candidiasis or to prepare the bowel for Gl surgery. Other anti-fungal agents that may be used according to the invention include, for example, Terazol, diflucan, butoconazole nitrate, Propulsid® (cisapride), and fluconazole. It should be noted that antimicrobial agents need not be present in the same composition of the invention as the beneficial bacteria. Rather, the antimicrobial agent may be introduced separately, before, after or concurrent with introduction of a composition comprising beneficial bactera to a subject. Dosages and treatment regimens using anti-fungal agents such as nystatin are established and available in the medical and pharmaceutical literature, and any permissible or recommended dosage may be used according to the invention. Dosing can be variable or individually prescribed by a physician or other medical professional. Dosages may be either high, intermediate or low dosages. Accordingly, in certain embodiments, nystatin dosages may be approximately 200,000 to 500,000 units twice daily for adults and children and approximately 100,000 units twice daily for infants.

### B. Probiotic Kits

In certain embodiments, compositions of the invention are provided in kits. Kits may include different types of compositions of the invention, generally depending on the reason the subject is being treated, the components included, and the method of delivery to the subject. Thus, in certain embodiments, kits include, for example, capsules or other encapsulated forms, tablets, and powders.

The kits may be used following an event that damages any system of the body normally containing beneficial bacteria, including the digestive and reproductive systems. Contemplated events include: use of antibiotics, birth control pills, chemotherapy, alcohol consumption, eating food with antibiotics contained in the food, drugs, or the ingestion of any substance that harms the gut environment or modifies the bacteria.

In different embodiments, kits comprise different components. In one embodiment, kits comprise at least two individually packed components. One component is the antifungal agent that may be a prescription drug. The other component is the bacteria and the bacteria nutrient. The bacteria nutrient may also be supplied as a third, individual component of a kit. The invention contemplates numerous classes of kits, each class containing different kits that may be administered by different means or to a different area of the subject's body. Examples of specific embodiments of kits include kits - formulated for post-antibiotic administration, kits designed specifically for colon care, kits designed to enhance the immune response, and kits designed for inoculation. Each kit may be taken orally or rectally and is available for use in humans and non-human animals.

Each kit contains one or more types of beneficial bacteria, typically specific for the condition being treated, and bacteria food and/or nutrients. In addition, the kits also contain an antifungal compound, for example, an anti-yeast agent such as nystatin. For example, nystatin is generally included in the post-antibiotic kits, the colon care kits, and the immune enhancing kits. Typically, each kit will comprise two distinct components: the antimicrobial agent and the probiotic component, which includes the bacteria and bacteria nutrients.

In one embodiment of the invention, a kit is provided for use following treatment with an antibiotic. The kit may be used before, during and/or following antibiotic use, although, preferably, the kit is taken between meals separately from the antibiotic itself. In one embodiment, the kit contains the bacteria Lactobacillus Acidophilus, Lactobacillus Bulgaricus, Streptococcus thermophilous and/or any symbiotic cofactors. Prior to administration, they may be mixed with one or more nutrients, such as spirilina and/or a blend of high lipid chloraphyllins, fructooligosaccharides and/or appropriate inulins, Methylsulfonylmethane (MSM) and ascorbic acid. In one embodiment, this mixture makes up one of two containers. The second container contains nystatin and/or another anti-fungal. In certain embodiments, the kit is designed for rectal administration and does not contain nystatin. In different embodiments, the kit is formulated for adults, infants or children by including different amounts of components based upon the appropriate dosage for the subject to be treated.

In another embodiment of the invention, kits are provided for colon care. Such kits are appropriate for a variety of diseases and disorders associated with disruption of bacterial flora of the colon, including, for example, diarrheal diseases, irritable bowel syndrome, cancer, diverticulitis, ostomies; ulcers, ulcerative colitis, and/or any bowel disorder or flu. In certain manifestations, the kit contains the bacteria Lactobacillus acidophilus, Streptococcus thermophilus, Lactobacillus bulgaricus, Bifidophilus longum, Bifidobacteria bifidus, and Bacillus laterosporus and/or other bacterial symbiotic cofactors, which are mixed with the food substance, spirilina, and/or a blend of high lipid chloraphyllins, fructooligosaccharides, and/or appropriate inulins. MSM with ascorbic acid are also included. This formula makes up one of two components. The second component comprises nystatin and/or another anti-fungal. The rectal form does not typically contain nystatin.

In another embodiment, the invention provides kits to assist the immune system and appropriate for administration to subjects suffering from a compromised immune system, including patients, for example, suffering from acquired immunodeficiency disease, cancer, high cholesterol, chronic disease of any kind, and any immune-compromising disease. This kit contains a broadest spectrum of bacteria, including Lactobacillus acidophilus, Bacillus bifidum, Lactobacillus bulgaricus, Lactobacillus plantarum, Lactobacillus rueteri, Streptococcus thermophilus, Lactobacillus salivarius, and Bifidobacteria bifidus. The bacteria are mixed with food or nutrient substance (*e.g*. spirilina and/or a blend of high lipid chloraphyllins, fructooligosaccrides and/or appropriate inulins), MSM with ascorbic acid. This formula makes up one of two components. The second component comprises nystatin and/or another anti-fungal. The rectal form does not typically contain nystatin.

In another embodiment, the invention provides kits for inoculating subjects with beneficial bacteria. Such kits are appropriate for use by anyone, whether or not they are sufferering from a disease or disorder or being treated with an antibiotic, for example. These kits may assist in fortifying the immune system against bio-terrorism attacks and is highly recommended for formula-fed infants. They may assist in protecting against flues and diarrheal symptoms, since most babies are not colonized adequately at birth. This kit is particularly valuable in third world countries and low-income areas where infant mortality is high due to diarrheal diseases and overuse and indiscriminate use of antibiotics. Ingredients in the kit formulated for infants include Bifodophilus infantis, fructooligosaccrides and/or appropriate inulins. These components may be mixed together in powder form, such that they can be added to formula or juice. Ingredients in the kit formulated for adults include the bacteria Lactobacillus acidophilus, Bacillus bifidum, Lactobacillus bulgaricus, Lactobacillus plantarum, Lactobacillus rueteri, Streptococcus thermophilus, Lactobacillus salivarius, and Bifidobacteria bifidus. These components are typically mixed with a bacteria nutrient or food substance (spirilina and/or a blend of high lipid chloraphyllins, fructooligosaccharides and/or appropriate inulins), MSM and ascorbic acid.

Kits may be formulated by a variety of methods, depending upon the contents and route of administration. In one embodiment, a kit is formulated by combining the beneficial bacteria (if more than one type of bacteria), combining spirilina and fructooligosaccharides with the resulting mixture, preparing a MSM mixture by combining ascorbic acid and MSM, and combining the MSM mixture with the bacteria and nutrient mixture. The resulting mixture may then be prepared for delivery, *e.g.* by being encapsulated in a gel capsule. In certain embodiments, any of the mixtures may be dried, *e.g.* lyophilized, to prepare a powder form. In another embodiment, the mixture is prepared in an aqueous medium for enema delivery.

### C. Probiotic Methods

The invention further provides for the use of the compositions and kits of the invention. Uses of the invention are fundamentally related to providing beneficial bacteria to a subject. Such uses may involve, for example, restoring beneficial bacteria reduced by treatment, for example, with antibiotics, chemotherapy, or radiation, combating infection by a pathogenic agent, such as, for example, a pathogenic bacteria, virus or other microbe, such as a yeast, or introducing beneficial bacteria to a subject, particularly a subject lacking normal of adequate amounts of beneficial bacteria, due, for example, to an inadequate food source for such bacteria.

Compositions and kits of the invention may be introduced to a subject via a variety of means, including orally, rectally, or vaginally, for example. Typically, the route of introduction is determined based on the location desired to be populated with beneficial bacteria and the nature of the disease or disorder being treated, if any. For example, treatment to prevent or reduce vaginal yeast infection resulting from antibiotic use may comprise introducing a composition of the invention vaginally, *e.g.* via a suppository. Compositions and kits of the invention may be introduced orally by any available means and in any available form, as known in the art. For example, the compositions may be introduced orally as a powder, *e.g*. to be mixed with food or drink, as a tablet or capsule, or as a liquid suspension. Compositions may also be introduced rectally or into the genitourinary tract in, *e.g*., suppositories, or liquid or cream suspensions, and as described supra.

Different components of the kits of the invention may be introduced together, *e.g*. as a mixture, at approximately the same time, *e.g*. within the same day or 24 hours, or at different times. Typically, the bacteria and bacteria nutrient will be introduced at the same time, and in certain embodiments, as a mixture. In certain embodiments, the bacteria are mixed and incubated with the nutrients prior to delivery to a subject. It is believed that such pre-mixing strengthens the bacteria for the environment of the gut or other area of the subject and facilitates colonization. The antimicrobial agent is -typically delivered separately from the bacteria and bacteria nutrient, although not necessarily. The antimicrobial agent may be delivered to the same or a different site than the bacteria and bacteria nutrient. For example, the antimicrobial may be delivered orally, while the bacteria and bacteria nutrient are delivered rectally, at approximately the same or a different time. The antimicrobial agent may be delivered prior to, at the same time, or subsequent to administration of the bacteria and bacteria nutrient.

Subjects that may be treated with the compositions, kits and methods of the invention include both human and non-human animals, including domestic animals and farm animals, for example. Human and non-human animals are treated routinely with antibiotics, which damages the normal intestinal flora. Subjects appropriate for treatment may be any age and include infants, children and adults.

In certain embodiments, subjects have been previously treated with or are being concurrently treated with an antibiotic. Kits and compositions of the invention may be introduced to a subject following or during treatment with any drug that depletes natural bioflora, such as an antibiotic. During the course of eliminating disease-causing bacteria, orally or systemically administered antibiotics will often destroy naturally-occurring beneficial bacterial flora. Diarrhea and yeast infections, including vaginal yeast, are common side-effects of the disruption of intestinal ecology. Examples of such drugs include aminoglycosides, amoxocillin, cephalosporins, ciprofloxacin, gentamicin, driseofulvin, neomycin, and tetracyclins.

Subjects also include humans and non-human animals suffering from a disease or disorder, particularly any disease or disorder that disrupts or otherwise reduces or effects the amount or type of bacterial flora present in the subject. Examples of diseases whose symptoms or condition may be improved or ameliorated using compositions or kits of the invention include, for example, cancer, acquired immunodeficiency disease, autoimmune disorders, food allergy, lactose intolerance, fatigue, malnutrition, neuropsychiatric symptoms, inflammatory bowel disease, ulcerative colitis, diarrheal diseases, diverticulitis, ostomies, ulcers, high cholesterol, chronic disease, immunocompromization, bacterial infection, yeast infection, viral infection, fungal infection, irritable bowel syndrome, Krohn's disease, periodontal disease, chronic respiratory or upper respiratory infection, ear infection, sinus infection, necrotizing enterocolitis, ileocecitis, multiple organ failure syndrome, pancreatitis, and burn injury.

In certain embodiments, subjects are suffering from an infection by any of a variety of pathological agents, including bacteria, yeast, or viruses, for example. Examples of microorganisms shown to be inhibited by L. acidophilus include, Bacillus subtilus, B. cereus, B. stearothermophilus, Candida albicans, Clostridium perfringens, Escheria coli, Klebsiella pneumoniae, L. bulgaricus, L. fermenti, L. helveticus, L. lactis, L. leichmannii, L. plantarum, Proteus vulgaricus, Pseudomonas aeruginosa, P. fluorescens, Salmonella typhosa, S. schottmuelleri, Shigella dysenteriae, S. paradysenteriae, Sarcina lutea, Serratia marcescens, Staphlococcus aureus, Streptococcus faecalis, S. lactis, and Vibrio comma.

## Claims

1. A kit comprising:
(i) bacteria, wherein the bacteria are selected from the group consisting of: Lactobacillus acidophilus, Lactobacillus bulgaricus, Streptococcus thermophilus, Bifidophilus longum, Bifidobacteria bifidus, Bacillus laterosporus, Bacillus bifidum, Lactobacillus plantarum, Lactobacillus rueteri, and Lactobacillus salivarius;
(ii) a bacteria nutrient, wherein the bacteria nutrient is selected from the group consisting of spirilina and/or a blend of high lipid chloraphyllins, fructooligosaccharides and/or inulins;
(iii) a methylsulfonylmethane (MSM) mixture, said mixture comprising MSM and ascorbic acid; and
(iv) an antimicrobial agent, wherein the antimicrobial agent is an anti-fungal compound.

2. The kit of claim 1,
wherein the bacteria comprise Lactobacillus acidophilus, Lactobacillus bulgaricus, and Streptococcus thermophilus, or
wherein the bacteria comprise Lactobacillus acidophilus, Streptococcus thermophilus, Lactobacillus bulgaricus, Bifidophilus longum, Bacillus laterosporus, or
wherein the bacteria comprise Lactobacillus acidophilus, Bacillus bifidum, Lactobacillus bulgaricus, Lactobacillus plantarum, Lactobacillus rueteri, Streptococcus thermophilus, Lactobacillus salivarius, and Bifidobacteria bifidus.

3. The kit of claim 1, wherein the antifungal compound is selected from the group consisting of nystatin, terazol, diflucan, butoconazole nitrate, Propulsid® (cisapride) and fluconazole.

4. A kit comprising Bifidophilus infantis and fructooligosaccharides, wherein these components are in powder form.

5. The kit of any of claims 1, to 4, wherein the bacteria is prepared by filtration.

6. The use of bacteria, wherein the bacteria are selected from the group consisting of: Lactobacillus acidophilus, Lactobacillus bulgaricus, Streptococcus thermophilus, Bifidophilus longum, Bifidobacteria bifidus, Bacillus laterosporus, Bacillus bifidum, Lactobacillus plantarum, Lactobacillus rueteri, and Lactobacillus salivarius, a bacteria nutrient, wherein the bacteria nutrient is selected from the group consisting of spirilina and/or a blend of high lipid chloraphyllins, fructooligosaccharides and/or inulins, a methylsulfonylmethane (MSM) mixture, said mixture comprising MSM and ascorbic acid, and an antimicrobial agent, wherein the antimicrobial agent is an anti-fungal compound, for manufacture of a pharmaceutical for enhancing bacteria in a patient.

7. The use of claim 6, wherein the patient is a human or a non-human animal.

8. The use of claim 6 or 7, wherein the bacteria and bacteria nutrient are introduced orally, wherein the bacteria and bacteria nutrient are introduced rectally, or wherein the bacteria and bacteria nutrient are introduced vaginally.

9. The use of claim 8, wherein the patient is also being treated with an antibiotic or wherein the patient is also being treated with radiation, a hormone, chemotherapy, ulcer medication, a steroid, or a vaccine.

10. The use of claim 6, wherein said patient is also treated with chemotherapy, radiation, or with an antibiotic.

11. The use of bacteria, wherein the bacteria are selected from the group consisting of: Lactobacillus acidophilus, Lactobacillus bulgaricus, Streptococcus thermophilus, Bifidophilus longum, Bifidobacteria bifidus, Bacillus laterosporus, Bacillus bifidum, Lactobacillus plantarum, Lactobacillus rueteri, and Lactobacillus salivarius, a bacteria nutrient, wherein the bacteria nutrient is selected from the group consisting of spirilina and/or a blend of high lipid chloraphyllins, fructooligosaccharides and/or inulins, a methylsulfonylmethane (MSM) mixture, said mixture comprising MSM and ascorbic acid, and an antimicrobial agent, wherein the antimicrobial agent is an anti-fungal compound, for manufacture of a pharmaceutical for inhibiting or reducing infection in a patient.

12. The use of claim 11, wherein the patient is suffering from a disease or condition selected from the group consisting of: cancer, acquired immunodeficiency disease, autoimmune disorders, food allergy, lactose intolerance, fatigue, malnutrition, neuropsychiatric symptoms, inflammatory bowel disease, ulcerative colitis, diarrheal diseases, diverticulitis, ostomies, ulcers, high cholesterol, chronic disease, immunocompromization, bacterial infection, yeast infection, viral infection, fungal infection, irritable bowel syndrome, Krohn's disease, periodontal disease, chronic respiratory or upper respiratory infection, ear infection, sinus infection, necrotizing enterocolitis, ileocolitis, multiple organ failure syndrome, pancreatitis, and burn injury.

13. The use of claim 6 or 11, wherein the antifungal compound is selected from the group consisting of nystatin, terazol, diflucan, butoconazole nitrate, Propuisid® (cisapride) and fluconazole.

## Patentansprüche

1. Ein Kit umfassend:
(i) Bakterien, wobei die Bakterien ausgewählt werden aus der Gruppe bestehend aus: Lactobacillus acidophilus, Lactobacillus bulgaricus, Streptococcus thermophilus, Bifidophilus longum, Bifidobacteria bifidus, Bacillus laterosporus, Bacillus bifidum, Lactobacillus plantarum, Lactobacillus rueteri, and Lactobacillus salivarius;
(ii) einen Bakterien-Nährstoff, wobei der Bakterien-Nährstoff ausgewählt wird aus der Gruppe bestehend aus Spirilina und/oder eine Mischung aus fettreichen Chlorophyllinen, Fructooligosacchariden und/oder Inulinen;
(iii) eine Methylsulfonylmethan (MSM) Mischung, wobei die Mischung MSM und Ascorbinsäure umfasst; und
(iv) einen antimikrobiellen Wirkstoff, wobei der antimikrobielle Wirkstoff ein Fungizid ist.

2. Das Kit nach Anspruch 1,
wobei die Bakterien Lactobacillus acidophilus, Lactobacillus bulgaricus, and Streptococcus thermophilus umfassen, oder
wobei die Bakterien Lactobacillus acidophilus, Streptococcus thermophilus, Lactobacillus bulgaricus, Bifidophilus longum, Bacillus laterosporus umfassen, oder
wobei die Bakterien Lactobacillus acidophilus, Bacillus bifidum, Lactobacillus bulgaricus, Lactobacillus plantarum, Lactobacillus rueteri, Streptococcus thermophilus, Lactobacillus salivarius, and Bifidobacteria bifidus umfassen.

3. Das Kit nach Anspruch 1, wobei das Fungizid ausgewählt wird aus der Gruppe bestehend aus Nystatin, Terazol, Diflucan, Butoconazolnitrat, Propulsid® (Cisaprid) und Fluconazol.

4. Ein Kit, das Bifidophilus infantis und Fructooligosaccharide umfasst, wobei diese Bestandteile in Pulverform vorliegen.

5. Das Kit nach einem der Ansprüche 1 bis 4, wobei die Bakterien durch Filtration gewonnen werden.

6. Die Verwendung von Bakterien, wobei die Bakterien ausgewählt werden aus der Gruppe bestehend aus: Lactobacillus acidophilus, Lactobacillus bulgaricus, Streptococcus thermophilus, Bifidophilus longum, Bifidobacteria bifidus, Bacillus laterosporus, Bacillus bifidum, Lactobacillus plantarum, Lactobacillus rueteri, und Lactobacillus salivarius,
einem Bakterien-Nährstoff, wobei der Bakterien-Nährstoff ausgewählt wird aus der Gruppe bestehend aus Spirilina und/oder einer Mischung aus fettreichen Chlorophyllinen, Fructooligosacchariden und/oder Inulinen,
einer Methylsulfonylmethan (MSM) Mischung, wobei die Mischung MSM und Ascorbinsäure umfasst, und
einem antimikrobiellen Wirkstoff, wobei der antimikrobielle Wirkstoff ein Fungizid ist,
zur Herstellung eines Arzneimittels zur Vermehrung von Bakterien in einem Patienten.

7. Die Verwendung nach Anspruch 6, wobei der Patient ein Mensch oder ein nichtmenschliches Tier ist.

8. Die Verwendung nach Anspruch 6 oder 7, wobei die Bakterien und der Bakterien-Nährstoff oral zugeführt werden, wobei die Bakterien und der Bakterien-Nährstoff rektal zugeführt werden oder wobei die Bakterien und der Bakterien-Nährstoff vaginal zugeführt werden.

9. Die Verwendung nach Anspruch 8, wobei der Patient auch mit einem Antibiotikum behandelt wird oder wobei der Patient auch mit Bestrahlung, einem Hormon, Chemotherapie, medikamentös wegen eines Geschwürs, mit einem Steroid, oder einem Impfstoff behandelt wird.

10. Die Verwendung nach Anspruch 6, wobei der Patient auch mit Chemotherapie, Bestrahlung oder mit einem Antibiotikum behandelt wird.

11. Die Verwendung von Bakterien, wobei die Bakterien ausgewählt werden aus der Gruppe bestehend aus: Lactobacillus acidophilus, Lactobacillus bulgaricus, Streptococcus thermophilus, Bifidophilus longum, Bifidobacteria bifidus, Bacillus laterosporus, Bacillus bifidum, Lactobacillus plantarum, Lactobacillus rueteri, und Lactobacillus salivarius,
einem Bakterien-Nährstoff, wobei der Bakterien-Nährstoff ausgewählt wird aus der Gruppe bestehend aus Spirilina und/oder einer Mischung aus fettreichen Chlorophyllinen, Fructooligosacchariden und/oder Inulinen,
einer Methylsulfonylmethan (MSM) Mischung, wobei die Mischung MSM und Ascorbinsäure umfasst, und
einem antimikrobiellen Wirkstoff, wobei der antimikrobielle Wirkstoff ein Fungizid ist,
zur Herstellung eines Arzneimittels zur Hemmung oder Abschwächung einer Infektion in einem Patienten.

12. Die Verwendung nach Anspruch 11, wobei der Patient unter einer Krankheit oder einem Erkrankung leidet, die/der ausgewählt wird aus der Gruppe bestehend aus: Krebs, erworbene Immunabwehrschwäche, Autoimmunerkrankung, Lebensmittelallergie, Laktose-Intoleranz, Erschöpfung, Fehlernährung, neuropsychiatrische Symptome, entzündliche Darmerkrankung, ulzerative Colitis, Durchfallerkrankungen, Divertikulitis, künstlicher Darmausgang, Geschwüre, erhöhte Cholesterinwerte, chronische Erkrankungen, Immunschwächung, bakterielle Infektion, Hefe Infektion, virale Infektion, Pilzinfektion, Reizdarm, Morbus Crohn, Erkrankung des Zahnfleisches, chronische Atemwegs- oder obere Atemwegsinfektion, Ohrinfektion, Nasennebenhöhleninfektion, nekrotisierende Enterocolitis, Darmverschlussentzündung, Multiorganversagen, Bauchspeicheldrüsenentzündung und Verbrennungsverletzung.

13. Die Verwendung nach Anspruch 6 oder 11, wobei das Fungizid ausgewählt wird aus der Gruppe bestehend aus Nystatin, Terazol, Diflucan, Butoconazolnitrat, Propulsid® (Cisaprid) und Fluconazol.

## Revendications

1. Kit comprenant :
(i) des bactéries, les bactéries étant choisies dans le groupe constitué de : *Lactobacillus acidophilus, Lactobacillus bulgaricus, Streptococcus thermophilus, Bifidophilus longum, Bifidobacteria bifidus, Bacillus laterosporus, Bacillus bifidum, Lactobacillus plantarum, Lactobacillus rueteri,* et *Lactobacillus salivarius* ;
(ii) un nutriment pour bactéries, le nutriment pour bactéries étant choisi dans le groupe constitué de la spiruline et/ou un mélange de chlorophyllines ayant une teneur élevée en lipides, de fructooligosaccharides et/ou d'inulines ;
(iii) un mélange de méthylsulfonylméthane (MSM), ledit mélange comprenant du MSM et de l'acide ascorbique ; et
(iv) un agent antimicrobien, ledit agent antimicrobien étant un composé antifongique.

2. Kit selon la revendication 1,
dans lequel les bactéries comprennent *Lactobacillus acidophilus, Lactobacillus bulgaricus,* et *Streptococcus thermophilus,* ou
dans lequel les bactéries comprennent *Lactobacillus acidophilus, Streptococcus thermophilus, Lactobacillus bulgaricus, Bifidophilus longum, Bacillus laterosporus,* ou
dans lequel les bactéries comprennent *Lactobacillus acidophilus, Bacillus bifidum, Lactobacillus bulgaricus, Lactobacillus plantarum, Lactobacillus rueteri, Streptococcus thermophilus, Lactobacillus salivarius,* et *Bifidobacteria bifidus.*

3. Kit selon la revendication 1, dans lequel le composé antifongique est choisi dans le groupe constitué de la nystatine, le terazol, le diflucan, le nitrate de butoconazole, le Propulsid^{®} (cisapride) et le fluconazole.

4. Kit comprenant *Bifidophilus infantis* et des fructooligosaccharides, dans lequel ces composants sont sous forme de poudre.

5. Kit selon l'une quelconque des revendications 1 à 4, dans lequel les bactéries sont préparées par filtration.

6. Utilisation de bactéries, les bactéries étant choisies dans le groupe constitué de : *Lactobacillus acidophilus, Lactobacillus bulgaricus, Streptococcus thermophilus, Bifidophilus longum, Bifidobacteria bifidus, Bacillus laterosporus, Bacillus bifidum, Lactobacillus plantarum, Lactobacillus rueteri,* et *Lactobacillus salivarius,* un nutriment pour bactéries, ledit nutriment pour bactéries étant choisi dans le groupe constitué de la spiruline et/ou un mélange de chlorophyllines ayant une teneur élevée en lipides, de fructooligosaccharides et/ou d'inulines, un mélange de méthylsulfonylméthane (MSM), ledit mélange comprenant du MSM et de l'acide ascorbique, et un agent antimicrobien, ledit agent antimicrobien étant un composé antifongique, dans la fabrication d'un produit pharmaceutique destiné à améliorer la flore bactérienne d'un patient.

7. Utilisation selon la revendication 6, dans laquelle le patient est un humain ou un animal non humain.

8. Utilisation selon la revendication 6 ou 7, dans laquelle les bactéries et le nutriment pour bactéries sont introduits par voie orale, dans laquelle les bactéries et le nutriment pour bactéries sont introduits par voie rectale, ou dans laquelle les bactéries et le nutriment pour bactéries sont introduits par voie vaginale.

9. Utilisation selon la revendication 8, dans laquelle le patient est également traité par un antibiotique ou dans laquelle le patient est également traité par radiation, une hormone, de la chimiothérapie, une médication contre les ulcères, un stéroïde, ou un vaccin.

10. Utilisation selon la revendication 6, dans laquelle ledit patient est également traité par de la chimiothérapie, de la radiation, ou un antibiotique.

11. Utilisation de bactéries, les bactéries étant choisies dans le groupe constitué de : *Lactobacillus acidophilus, Lactobacillus bulgaricus, Streptococcus thermophilus, Bifidophilus longum, Bifidobacteria bifidus, Bacillus laterosporus, Bacillus bifidum, Lactobacillus plantarum, Lactobacillus rueteri,* et *Lactobacillus salivarius,* un nutriment pour bactéries, le nutriment pour bactéries étant choisis dans le groupe constitué de la spiruline et/ou un mélange de chlorophyllines ayant une teneur élevée en lipides, de fructooligosaccharides et/ou d'inulines, un mélange de méthylsulfonylméthane (MSM), ledit mélange comprenant du MSM et de l'acide ascorbique, et un agent antimicrobien, ledit agent antimicrobien étant un composé antifongique, dans la fabrication d'un produit pharmaceutique destiné à inhiber ou à réduire une infection chez un patient.

12. Utilisation selon la revendication 11, dans laquelle le patient souffre d'une maladie ou d'un état pathologique choisi(e) dans le groupe constitué d'un cancer, une maladie liée à un déficit immunitaire acquis, des troubles auto-immuns, une allergie alimentaire, une intolérance au lactose, de la fatigue, de la malnutrition, des symptômes neuropsychiatriques, une maladie inflammatoire de l'intestin, une rectocolite hémorragique, des maladies diarrhéiques, la diverticulite, des stomies, des ulcères, un taux élevé de cholestérol, une maladie chronique, une immunodéficience, une infection bactérienne, une infection provoquée par des levures, une infection virale, une infection fongique, le syndrome du côlon irritable, la maladie de Crohn, une maladie parodontale, une infection respiratoire chronique ou une infection des voies respiratoires supérieures, une infection de l'oreille, une infection des sinus, une entérocolite nécrosante, une iléocolite, le syndrome de défaillance multiviscérale, une pancréatite, et une brûlure.

13. Utilisation selon la revendication 6 ou 11, dans laquelle le composé antifongique est choisi dans le groupe constitué de la nystatine, le terazol, le diflucan, le nitrate de butoconazole, le Propulsid^{®} (cisapride) et le fluconazole.
